# EUROPEAN PATENT APPLICATION

(11) **EP 4 212 099 A1**
(43) Date of publication of application: **19.07.2023**
(21) Application number: 21940901.8
(22) Date of filing: 22.12.2021
(51) Int. Cl.: A61B 5/361

(54) **PROGRAM, OUTPUT DEVICE, AND DATA PROCESSING METHOD**

(30) Priority: 21.05.2021 JP 2021085848
(71) Applicant: Cardio Intelligence Inc., Minato-ku Tokyo 106-0044 (JP)
(72) Inventor: TAMURA, Yuichi, Tokyo 106-0044 (JP); TAKATA, Tomohiro, Tokyo 106-0044 (JP); TANIGUCHI, Hirohisa, Tokyo 106-0044 (JP)
(74) Representative: Plougmann Vingtoft a/s
(86) International application number: PCT/JP2021/047643
(87) International publication number: WO 2022/244291

(57) **Abstract**

A program for causing a computer to execute the steps of: acquiring one or more pieces of fibrillation wave electrocardiogram data including fibrillation waves included in whole electrocardiogram data obtained by measuring change over time of an action potential due to electric activity of a heart muscle; inputting the one or more pieces of fibrillation wave electrocardiogram data into a first machine learning model that classifies a plurality of pieces of electrocardiogram data including the fibrillation waves into electrocardiogram data in which the fibrillation waves are clear and electrocardiogram data in which the fibrillation waves are unclear, and acquiring clear electrocardiogram data output from the first machine learning model as the electrocardiogram data in which the fibrillation waves are clear; and outputting the clear electrocardiogram data.

## Description

### TECHNICAL FIELD

The present disclosure relates to a program, an output apparatus, and a data processing method for analyzing an electrocardiogram.

### BACKGROUND OF THE INVENTION

Examining a long-term electrocardiogram may be required for making a diagnosis of heart disease such as paroxysmal atrial fibrillation. Since the electrocardiogram necessitates approximately 100,000 waveforms in 24 hours, it is a heavy burden to review the long-term electrocardiogram, especially for a doctor who is not a cardiovascular specialist. Patent Document 1 describes that monitored electrocardiogram (ECG) samples are flagged for urgent review by a human expert.

### PRIOR ART

### PATENT DOCUMENT

[Patent Document 1] Japanese Unexamined Patent Application Publication (Translation of PCT Application) No. 2019-502426

### SUMMARY OF INVENTION

### PROBLEMS TO BE SOLVED BY THE INVENTION

In the technology described in Patent Document 1, electrocardiogram samples flagged for urgent review are communicated to a cardiovascular specialist for review. However, these electrocardiogram samples may differ from electrocardiogram examples showing typical features of heart disease. For this reason, there were cases where a doctor who is not a cardiovascular specialist cannot use these electrocardiogram samples as the basis for determining heart disease.

The present disclosure focuses on this point and its object is to provide a program, an output apparatus, and a data processing method capable of enabling a doctor to grasp a waveform of an electrocardiogram which is the basis for the doctor to determine that a patient has heart disease.

### MEANS FOR SOLVING THE PROBLEMS

A program according to a first aspect of the present disclosure is a program for causing a computer to execute the steps of acquiring one or more pieces of fibrillation wave electrocardiogram data including fibrillation waves included in whole electrocardiogram data obtained by measuring change over time of an action potential caused by electric activity of a heart muscle; inputting the one or more pieces of fibrillation wave electrocardiogram data into a first machine learning model that classifies a plurality of pieces of electrocardiogram data including the fibrillation waves into (i) electrocardiogram data in which the fibrillation waves are clear and (ii) electrocardiogram data in which the fibrillation waves are unclear, and acquiring clear electrocardiogram data output from the first machine learning model as the electrocardiogram data in which the fibrillation waves are clear; and outputting the clear electrocardiogram data.

The acquiring the one or more pieces of fibrillation wave electrocardiogram data may include: generating a plurality of pieces of divided electrocardiogram data obtained by dividing the whole electrocardiogram data; inputting the generated plurality of pieces of divided electrocardiogram data to a second machine learning model that classifies the plurality of pieces of electrocardiogram data into (i) the electrocardiogram data including fibrillation waves and (ii) the electrocardiogram data not including fibrillation waves; and acquiring one or more pieces of fibrillation wave electrocardiogram data output as the electrocardiogram data including the fibrillation waves output from the second machine learning model.

The outputting may include outputting the clear electrocardiogram data in a state in which image data indicating that fibrillation waves are included is superimposed on a time domain between a plurality of R waves in the clear electrocardiogram data. The outputting may include outputting the clear electrocardiogram data in which image data indicating that fibrillation waves are included is superimposed on a time domain within a predetermined time period before a timing at which a Q wave starts in the clear electrocardiogram data. The outputting may include outputting the clear electrocardiogram data in which the image data indicating that the fibrillation waves are included is superimposed on a time domain between a timing at which a T wave ends and a timing at which the Q wave starts in the clear electrocardiogram data.

The program may further include determining whether there is a signal in a frequency range corresponding to fibrillation waves by performing a frequency analysis on the acquired clear electrocardiogram data, wherein the outputting may include outputting the clear electrocardiogram data for which the determining determined that the signal in the frequency range corresponding to the fibrillation waves exists therein.

An output apparatus according to a second aspect of the present disclosure including: a first acquisition part that acquires one or more pieces of fibrillation wave electrocardiogram data including fibrillation waves included in whole electrocardiogram data obtained by measuring change over time of an action potential caused by electric activity of a heart muscle; and a second acquisition part that inputs the one or more pieces of fibrillation wave electrocardiogram data to a first machine learning model that classifies a plurality of pieces of electrocardiogram data including the fibrillation waves into (i) electrocardiogram data in which the fibrillation waves are clear and (ii) electrocardiogram data in which the fibrillation waves are unclear, and acquires clear electrocardiogram data output from the first machine learning model as the clear electrocardiogram data in which the fibrillation waves are clear; and an output part that outputs the clear electrocardiogram data acquired by the second acquisition part.

A data processing method according to a third aspect of the present disclosure is a data processing including the computer-implemented steps of: acquiring one or more pieces of fibrillation wave electrocardiogram data including fibrillation waves included in whole electrocardiogram data obtained by measuring change over time of an action potential caused by electric activity of a heart muscle; inputting the one or more pieces of fibrillation wave electrocardiogram data into a first machine learning model that classifies a plurality of pieces of electrocardiogram data including the fibrillation waves into (i) electrocardiogram data in which the fibrillation waves are clear and (ii) electrocardiogram data in which the fibrillation waves are unclear, and acquiring clear electrocardiogram data output from the first machine learning model as the electrocardiogram data in which the fibrillation waves are clear; and outputting the clear electrocardiogram data.

The data processing method further including, prior to acquiring the clear electrocardiogram data: acquiring a plurality of pieces of learning electrocardiogram data which are a plurality of pieces of electrocardiogram data including fibrillation waves; accepting an instruction to classify the plurality of pieces of learning electrocardiogram data into clear learning electrocardiogram data in which fibrillation waves are clear and unclear learning electrocardiogram data in which the fibrillation waves are unclear; and generating a machine learning model that classifies a plurality of pieces of electrocardiogram data including the fibrillation waves into (i) electrocardiogram data in which the fibrillation waves are clear and (ii) electrocardiogram data in which the fibrillation waves are unclear by learning a plurality of pieces of the clear learning electrocardiogram data and a plurality of pieces of the unclear learning electrocardiogram data, both serving as training data.

### EFFECT OF THE INVENTION

According to the present disclosure, it is possible for a doctor to grasp a waveform of an electrocardiogram, which is the basis for the doctor to determine that a patient has heart disease.

### BRIEF DESCRIPTION OF THE DRAWINGS

FIG. 1 illustrates an outline of an electrocardiogram output system according to an embodiment.
FIG. 2 shows a configuration of an output apparatus.
FIG. 3 shows an example of whole electrocardiogram data and divided electrocardiogram data.
FIG. 4 shows an example of an electrocardiogram including fibrillation waves.
FIG. 5 shows an example of outputting clear electrocardiogram data with an output part.
FIG. 6 is a flowchart showing a processing procedure for generating a machine learning model for display, performed by the output apparatus.
FIG. 7 is a flowchart showing a processing procedure for outputting the clear electrocardiogram data, performed by the output apparatus.

### DETAILED DESCRIPTION OF THE INVENTION

### [Outline of an electrocardiogram display system S]

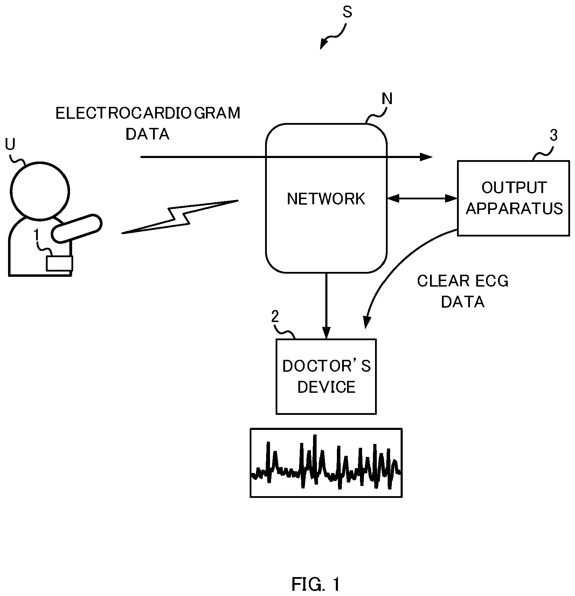

FIG. 1 illustrates an outline of an electrocardiogram output system S according to the present embodiment. The electrocardiogram output system S is a system for making it easy for a doctor to diagnose a patient by using an electrocardiogram of a patient U who may have atrial fibrillation. The electrocardiogram display system S includes an electrocardiograph 1, a doctor's device 2, and an output apparatus 3.

The electrocardiograph 1 is a Holter monitor worn by the patient U, for example. The electrocardiograph 1 generates whole electrocardiogram data obtained by measuring the time variation of an action potential caused by the electric activity of the heart muscle of the patient U. The electrocardiograph 1 transmits the generated whole electrocardiogram data to the output apparatus 3 via a network N including a wireless communication line. Time information indicating a measurement timing of the whole electrocardiogram data is associated with the whole electrocardiogram data. The whole electrocardiogram data generated by the electrocardiograph 1 may be sent to the output apparatus 3 using a storage medium without passing through the network N, for example.

The doctor's device 2 is a terminal used by a doctor, and includes a display and a computer, for example. The doctor's device 2 outputs, to the display, a waveform image based on a part of the electrocardiogram data received from the output apparatus 3 within the whole electrocardiogram data generated by the electrocardiograph 1.

The output apparatus 3 is a server, for example. The output apparatus 3 outputs information that supports a diagnosis by a doctor on whether atrial fibrillation has occurred in the heart of the patient U. The output apparatus 3 receives the whole electrocardiogram data of the patient U from the electrocardiograph 1 or the doctor's device 2. The output apparatus 3 generates a plurality of pieces of divided electrocardiogram data obtained by dividing the received whole electrocardiogram data. The output apparatus 3 acquires a plurality of pieces of fibrillation wave electrocardiogram data including fibrillation waves specific to atrial fibrillation, within the plurality of pieces of divided electrocardiogram data.

The output apparatus 3 reads, from the storage part, a machine learning model for display (corresponding to a first machine learning model) that has learned to classify a plurality of pieces of electrocardiogram data including fibrillation waves into (i) electrocardiogram data whose fibrillation waves are clear and (ii) electrocardiogram data whose fibrillation waves are unclear. An internal configuration of the machine learning model is any desired configuration, and it includes a Convolutional Neural Network (CNN), for example.

The output apparatus 3 inputs the acquired plurality of pieces of fibrillation wave electrocardiogram data to the learned machine learning model for display, and acquires clear electrocardiogram data, which is output by the machine learning model for display as the electrocardiogram data. The output apparatus 3 outputs the acquired clear electrocardiogram data to the doctor's device 2. In this manner, the output apparatus 3 can enable the doctor to grasp the waveform of the electrocardiogram indicating a basis for the doctor to determine that atrial fibrillation has occurred in the heart of the patient U.

Further, the output apparatus 3 is not limited to an example in which it is a computer different from the doctor's device 2. For example, the output apparatus 3 may be the same computer as the doctor's device 2. Hereinafter, the configuration and operation of the output apparatus 3 will be described in detail.

### [Configuration of the output apparatus 3]

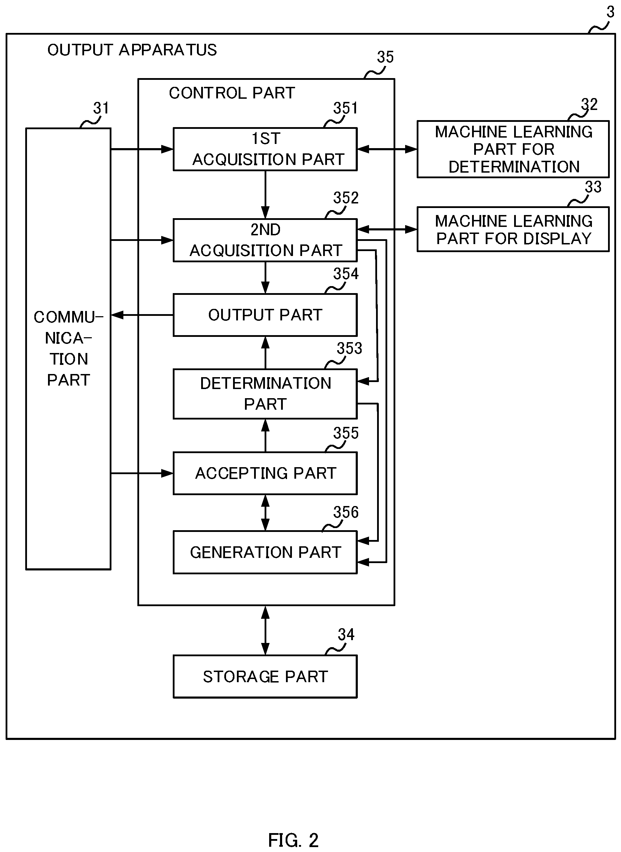

FIG. 2 shows the configuration of the output apparatus 3. The output apparatus 3 includes a communication part 31, a machine learning part 32 for determination, a machine learning part 33, a machine learning part 33 for display, a storage part 34, and a control part 35. The control part 35 includes a first acquisition part 351, a second acquisition part 352, a determination part 353, an output part 354, an accepting part 355, and a generation part 356.

The communication part 31 includes a communication controller for transmitting and receiving data between the electrocardiograph 1 and the doctor's device 2 via the network N. The communication part 31 notifies the control part 35 about data received via the network N.

The machine learning part 32 for determination functions as a determination machine learning model (corresponding to a second machine learning model) that can classify the inputted plurality of pieces of electrocardiogram data into (i) electrocardiogram data including fibrillation waves and (ii) electrocardiogram data not including fibrillation waves by learning on the basis of learning electrocardiogram data, which is to be used as training data. The machine learning part 32 for determination includes a processor that executes various calculations using the CNN, and a memory that stores coefficients of the CNN, for example. The machine learning part 32 for determination classifies the inputted electrocardiogram data into the electrocardiogram data including fibrillation waves and the electrocardiogram data not including fibrillation waves, and outputs them.

By learning on the basis of the learning electrocardiogram data, used as the training data, the machine learning part 33 for display functions as the above-described machine learning model for display that can classify the inputted plurality of pieces of electrocardiogram data including fibrillation waves into (i) electrocardiogram data whose fibrillation waves are clear and (ii) electrocardiogram data whose fibrillation waves are unclear. The machine learning part 33 for display includes a processor that performs various calculations using a CNN and a memory that stores coefficients of the CNN, for example. The machine learning part 33 for display classifies the inputted plurality of pieces of electrocardiogram data including fibrillation waves into (i) the electrocardiogram data whose fibrillation waves are clear and (ii) the electrocardiogram data whose fibrillation waves are unclear, and outputs them.

The storage part 34 includes a storage medium such as read only memory (ROM), a random access memory (RAM), and a hard disk. The storage part 34 stores a program executed by the control part 35. The storage part 34 stores various types of data necessary for the control part 35 to execute various calculations.

The control part 35 is a processor such as a central processing unit (CPU), for example. By executing the program stored in the storage part 34, the control part 35 functions as the first acquisition part 351, the second acquisition part 352, the determination part 353, the output part 354, the accepting part 355, and the generation part 356.

The first acquisition part 351 communicates with the electrocardiograph 1 via the communication part 31. The first acquisition part 351 acquires, from the electrocardiograph 1, whole electrocardiogram data obtained by measuring the time variation of an action potential caused by the electric activity of the heart muscle. The first acquisition part 351 acquires one or more pieces of fibrillation wave electrocardiogram data including fibrillation waves within the electrocardiogram data included in the acquired whole electrocardiogram data. First, the first acquisition part 351 divides the acquired whole electrocardiogram data into a plurality of pieces of divided electrocardiogram data. For example, the first acquisition part 351 generates a plurality of pieces of divided electrocardiogram data obtained by dividing the whole electrocardiogram data.

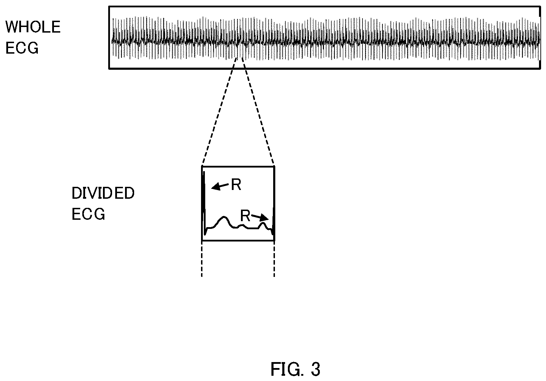

FIG. 3 shows an example of the whole electrocardiogram data and the divided electrocardiogram data. The upper side shows the whole electrocardiogram included in the whole electrocardiogram data, and the lower side shows a divided electrocardiogram included in the divided electrocardiogram data. The whole electrocardiogram shows a measurement result of measuring the change over time in the action potential of the heart muscle of the patient U over a predetermined measurement time. The measurement time is 24 hours, for example. The divided electrocardiogram data is obtained by dividing the whole electrocardiogram data. For example, the whole electrocardiogram data is divided at each rising point at which the potential indicated by the whole electrocardiogram data goes from below or equal to a threshold value to above the threshold value. The threshold value is a value that is assumed to be lower than a value assumed for a peak other than an R wave and higher than a peak of a wave other than the R wave, for example. The divided electrocardiogram data may be obtained by dividing the whole electrocardiogram data into predetermined time lengths. The predetermined time length is a time corresponding to one or several cycles of the R wave included in the electrocardiogram data, for example. "R" at the right end and "R" at the left end in the divided electrocardiogram of FIG. 3, represent a part of the R wave.

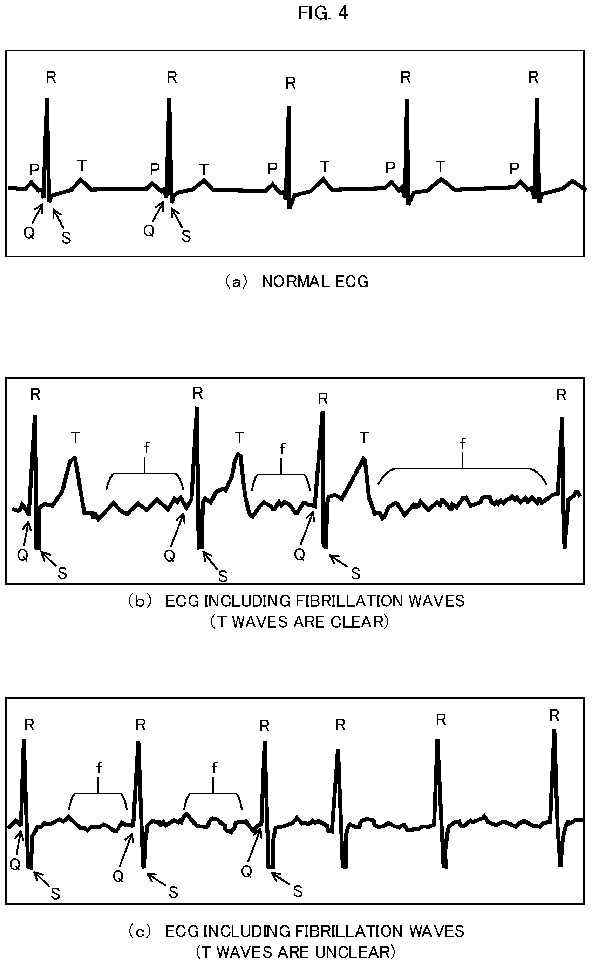

The first acquisition part 351 acquires one or more pieces of fibrillation wave electrocardiogram data including fibrillation waves, among the plurality of pieces of divided electrocardiogram data. FIGS. 4A to 4C each show an example of an electrocardiogram including fibrillation waves. FIG. 4A shows a normal electrocardiogram. FIG. 4B shows an example of an electrocardiogram including fibrillation waves in which T waves are clear. FIG. 4C shows an example of an electrocardiogram including fibrillation waves in which the T waves are not clear. The vertical axis in FIG. 4A represents a potential, and the horizontal axis in FIG. 4A represents time. "P", "Q", "R", "S", and "T" in FIG. 4A respectively denote a P wave, a Q wave, an R wave, an S wave, and a T wave. As shown in FIG. 4A, in the normal electrocardiogram, the P wave, the Q wave, the R wave, the S wave, and the T wave are each repeated at a fixed period. The normal electrocardiogram shown in FIG. 4A includes no fibrillation waves.

"f' in FIG. 4B denotes fibrillation waves. In the example of FIG. 4B, it is understood that atrial fibrillation occurs in the heart of the patient U since the electrocardiogram includes fibrillation waves. In the electrocardiogram of a state in which atrial fibrillation occurs, disappearance of the P wave is observed and the periods of the R wave and the like become irregular, as shown in FIG. 4B. In the example of FIG. 4B, clear T waves are seen. FIG. 4C shows, similarly to FIG. 4B, an example of the electrocardiogram including fibrillation waves. The example of FIG. 4C differs from the example of FIG. 4B in that the T waves are not clear. Disappearance of the P wave is also observed in the electrocardiogram shown in FIG. 4C and the periods of the R wave and the like become irregular.

In the example of the present specification, the first acquisition part 351 inputs a plurality of pieces of divided electrocardiogram data to the machine learning part 32 for determination functioning as the determination machine learning model (corresponding to the second machine learning model), and acquires one or more pieces of fibrillation wave electrocardiogram data including fibrillation waves output as the electrocardiogram data including fibrillation waves from the machine learning part 32 for determination. The first acquisition part 351 outputs the acquired one or more pieces of fibrillation wave electrocardiogram data to the second acquisition part 352.

### [Acquisition of the electrocardiogram data whose fibrillation waves are clear]

The second acquisition part 352 acquires clear electrocardiogram data whose fibrillation waves are clear, among the one or more pieces of fibrillation wave electrocardiogram data acquired by the first acquisition part 351. In the example of the present specification, the second acquisition part 352 inputs one or more pieces of fibrillation wave electrocardiogram data to the machine learning part 33 for display that functions as the machine learning model for display, and acquires clear electrocardiogram data which is output as the clear electrocardiogram data whose fibrillation waves are clear from the machine learning part 33 for display.

At this time, instead of inputting the fibrillation wave electrocardiogram data to the machine learning part 33 for display, the second acquisition part 352 may input modified fibrillation wave electrocardiogram data, obtained by removing waves different from the fibrillation waves from the fibrillation wave electrocardiogram data, to the machine learning part 33 for display. For example, the second acquisition part 352 generates the modified fibrillation wave electrocardiogram data by removing one or more of the P wave, the Q wave, the R wave, the S wave, and the T wave (see FIGS. 4A to 4C). The second acquisition part 352 inputs the generated one or more pieces of modified fibrillation wave electrocardiogram data to the machine learning part 33 for display.

For example, the second acquisition part 352 generates the modified fibrillation wave electrocardiogram data by removing a wave having a potential equal to or higher than a reference value from the waveform of the electrocardiogram including fibrillation waves. The reference value is higher than a value assumed to be a potential of the peak of the fibrillation waves, for example. Further, the second acquisition part 352 may identify time domains in which the Q wave, the R wave, the S wave, the T wave, and the like occurred, and may generate the modified fibrillation wave electrocardiogram data from which waves other than the fibrillation waves are removed, by setting the potential of the electrocardiogram to zero in these time domains.

For example, the second acquisition part 352 identifies a time domain in which the potential is equal to or greater than the threshold value as a time domain in which the R wave occurred. Time domains in which the Q wave, the S wave, and the T wave occurred can be estimated from time domains in which preceding and following R waves occurred. The second acquisition part 352 identifies the respective time domains in which the Q wave, the S wave, and the T wave occurred on the basis of the identified plurality of time domains in which the R waves occurred. The second acquisition part 352 may generate the modified fibrillation wave electrocardiogram data by setting the potential of the electrocardiogram to zero in each time domain in which the identified Q wave, R wave, S wave, T wave, and the like occurred.

The second acquisition part 352 inputs one or more pieces of modified fibrillation wave electrocardiogram data obtained by removing the waves different from the fibrillation waves to the machine learning part 33 for display, and acquires modified fibrillation wave electrocardiogram data output from the machine learning part 33 for display as the electrocardiogram data whose fibrillation waves are clear. The second acquisition part 352 may acquire, on the basis of the modified fibrillation wave electrocardiogram data output as the clear electrocardiogram data whose fibrillation waves are clear, fibrillation wave electrocardiogram data before removing the waves different from the fibrillation waves in said modified fibrillation wave electrocardiogram data, as the clear electrocardiogram data whose fibrillation waves are clear.

The determination part 353 determines whether there is a signal in a frequency range corresponding to the fibrillation waves by performing a frequency analysis on the clear electrocardiogram data acquired by the second acquisition part 352. The frequency analysis is a Fourier analysis or a wavelet analysis, for example. The determination part 353 notifies the output part 354 about a determination result of whether there is the signal in the frequency range corresponding to the fibrillation waves.

### [Output of the electrocardiogram data]

The output part 354 communicates with the doctor's device 2 via the communication part 31. The output part 354 outputs the clear electrocardiogram data acquired by the second acquisition part 352. For example, the output part 354 outputs the clear electrocardiogram data to the display of the doctor's device 2. The output part 354 outputs the clear electrocardiogram data in a state in which image data indicating that the fibrillation waves are included is superimposed on a time domain between the plurality of R waves in the clear electrocardiogram data acquired by the second acquisition part 352.

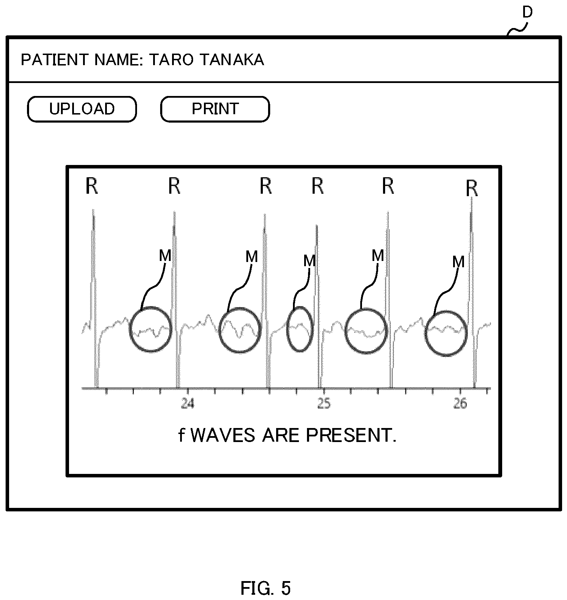

FIG. 5 shows an example of outputting the clear electrocardiogram data by the output part 354. An image shown in FIG. 5 is output to a display D of the doctor's device 2. In the electrocardiogram of FIG. 5, "R" denotes the R wave. The output part 354 displays image data M indicating that fibrillation waves are included in time domains between the plurality of R waves. In the example of FIG. 5, a thick elliptical frame is shown as the image data M.

In a state where atrial fibrillation occurs, the period of the R wave increases or decreases irregularly. Therefore, in the example of FIG. 5, the output part 354 displays the image data M with a size in the time direction varies depending on the period of the R wave. For example, when the period of the R wave is smaller than a predetermined value, the output part 354 displays the image data M with a size in the time direction is a first size. When the period of the R wave is equal to or greater than the predetermined value, the output part 354 displays the image data M with a size in the time direction is a second size. The second size is larger than the first size. In the example of FIG. 5, the output part 354 outputs the image data M together with a message "f waves are present", indicating that the image data M corresponds to the position of fibrillation waves.

In a state in which atrial fibrillation occurs, the fibrillation waves exist in almost any time domain of the electrocardiogram. However, the fibrillation waves are not clear at a timing when the fibrillation waves overlap with other waves such as Q, R, S, and T waves. In particular, the fibrillation waves often become distinct at a position between the plurality of R waves, since the R waves have higher peak potentials than the other waves. Therefore, the output part 354 outputs the clear electrocardiogram data in a state in which the image data M indicating that the fibrillation waves are included is superimposed on time domains between the plurality of R waves. As an example, the output part 354 outputs the clear electrocardiogram data in a state in which the image data M is superimposed on a time domain including an intermediate position of the plurality of R waves. By doing this, the output part 354 can make it easier for the doctor to grasp a time domain that includes clear fibrillation waves.

In a state where atrial fibrillation occurs, disappearance of the P wave is observed in the electrocardiogram. Normally, since the P wave does not occur at the same time as the R wave and the T wave, the R wave and the T wave do not occur in a time domain in which the P wave has disappeared. Therefore, the fibrillation waves become distinct in this time domain.

The output part 354 may identify a time domain in which the P wave should occur if atrial fibrillation has not occurred, and display the image data M indicating that clear fibrillation waves are included in the identified time domain. Since the P wave occurs before the Q wave, the output part 354 may output clear electrocardiogram data in a state in which the image data M is superimposed on a time domain within a predetermined time period before the timing at which the Q wave starts. The predetermined time period is determined such that the image data M includes a P-wave domain if the P wave has not disappeared, for example. Further, in the normal electrocardiogram data in which atrial fibrillation does not occur, the P wave occurs between a timing at which the T wave ends and a timing at which the Q wave starts (see FIG. 4A). Therefore, the output part 354 may identify the positions of the T wave and the Q wave in the electrocardiogram data, and may output the clear electrocardiogram data in a state in which the image data M is superimposed on a time domain between the timing at which the T wave ends and the timing at which the Q wave starts.

Further, among the clear electrocardiogram data acquired by the second acquisition part 352, the output part 354 may output the clear electrocardiogram data determined by the determination part 353 that the signal in the frequency range corresponding to the fibrillation waves exists therein. Among the clear electrocardiogram data acquired by the second acquisition part 352, the output part 354 need not output the clear electrocardiogram data determined by the determination part 353 to have no signal in the frequency range corresponding to the fibrillation waves. In this way, if the second acquisition part 352 erroneously acquires clear electrocardiogram data that does not include the fibrillation waves, the output part 354 can prevent the output of said clear electrocardiogram data.

### [Processing at the time of generation of the machine learning model for display]

With reference to FIG. 2 again, processing at the time of machine learning for generating the machine learning model for display will be described as a process before the second acquisition part 352 acquires the clear electrocardiogram data. The first acquisition part 351 acquires a plurality of pieces of learning electrocardiogram data which are a plurality of pieces of electrocardiogram data including fibrillation waves. At this time, the first acquisition part 351 acquires a plurality of pieces of whole electrocardiogram data from electrocardiographs 1 respectively worn by a plurality of users U. The first acquisition part 351 acquires a plurality of pieces of learning electrocardiogram data including fibrillation waves among the acquired plurality of pieces of whole electrocardiogram data by using the machine learning part 32 for determination that classifies the plurality of pieces of electrocardiogram data into the electrocardiogram data including fibrillation waves and the electrocardiogram data not including fibrillation waves. Specifically, the first acquisition part 351 inputs the whole electrocardiogram data to the determination machine learning model, and acquires the plurality of pieces of electrocardiogram data including fibrillation waves output from the determination machine learning model as the plurality of pieces of learning electrocardiogram data.

The accepting part 355 communicates with the doctor's device 2 via the communication part 31. The accepting part 355 accepts an instruction to classify the plurality of pieces of learning electrocardiogram data into clear learning electrocardiogram data whose fibrillation waves are clear and unclear learning electrocardiogram data whose fibrillation waves are unclear. More specifically, the accepting part 355 sequentially outputs the plurality of pieces of learning electrocardiogram data acquired by the first acquisition part 351 to the display of the doctor's device 2. The accepting part 355 accepts, for each piece of learning electrocardiogram data, a doctor's instruction to classify the output learning electrocardiogram data into either the clear learning electrocardiogram data with clear fibrillation waves or the unclear learning electrocardiogram data with unclear fibrillation waves, from the doctor's device 2. The accepting part 355 labels each of the plurality of pieces of learning electrocardiogram data with the classification result made by the doctor as to whether it is clear learning electrocardiogram data with clear fibrillation waves or unclear learning electrocardiogram data with unclear fibrillation waves, and causes the storage part 34 to store them.

The determination part 353 may determine whether there is the signal in the frequency range corresponding to the fibrillation waves by performing a frequency analysis on the plurality of pieces of learning electrocardiogram data acquired by the first acquisition part 351. The determination part 353 may delete, from the storage part 34, the learning electrocardiogram data determined not to include a signal in the frequency range corresponding to the fibrillation waves. In this way, the determination part 353 can prevent the learning electrocardiogram data determined not to include a signal in the frequency range corresponding to the fibrillation waves from being used for machine learning by the generation part 356.

The generation part 356 generates a machine learning model for display, which classifies a plurality of pieces of electrocardiogram data including fibrillation waves into the electrocardiogram data whose fibrillation waves are clear and the electrocardiogram data whose fibrillation waves are unclear. The generation part 356 generates the machine learning model for display by machine-learning a plurality of pieces of learning electrocardiogram data labeled as the clear learning electrocardiogram data and a plurality of pieces of learning electrocardiogram data labeled as the unclear learning electrocardiogram data, serving as training data. In this way, the generation part 356 can generate the above-described machine learning model for display. When generating the machine learning model for display, the electrocardiogram display system S transmits the plurality of pieces of electrocardiogram data including fibrillation waves output from the determination machine learning model to a doctor who checks whether or not the fibrillation waves are clear. Therefore, the number of pieces of electrocardiogram data to be checked by the doctor can be narrowed down. Therefore, the electrocardiogram display system S can generate the machine learning model for display in a short period of time.

### [Processing procedure for generating the machine learning model for display]

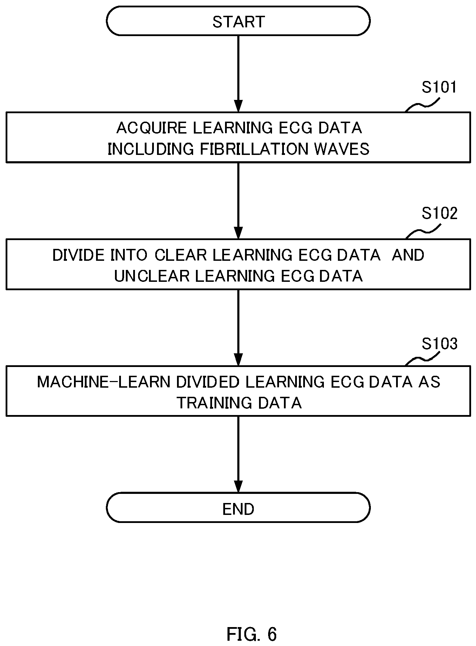

FIG. 6 is a flowchart showing a processing procedure for generating the machine learning model for display, performed by the output apparatus 3. This processing procedure starts when the accepting part 355 accepts an instruction to generate a machine learning model for display from the doctor's device 2, for example.

The first acquisition part 351 acquires a plurality of pieces of learning electrocardiogram data which are a plurality of pieces of electrocardiogram data including fibrillation waves (S101). The accepting part 355 accepts, from the doctor's device 2, an instruction to classify the plurality of pieces of learning electrocardiogram data into clear learning electrocardiogram data whose fibrillation waves are clear and unclear learning electrocardiogram data whose fibrillation waves are unclear (S102). The accepting part 355 labels each of the plurality of pieces of learning electrocardiogram data with a classification result classified by a doctor as to whether it is clear learning electrocardiogram data with clear fibrillation waves or unclear learning electrocardiogram data with unclear fibrillation waves, and causes the storage part 34 to store them. The generation part 356 generates a machine learning model for display by machine-learning a plurality of pieces of learning electrocardiogram data labeled as clear learning electrocardiogram data and a plurality of pieces of learning electrocardiogram data labeled as unclear learning electrocardiogram data, both serving as training data (S103), and ends the process.

### [Processing procedure for outputting the clear electrocardiogram data]

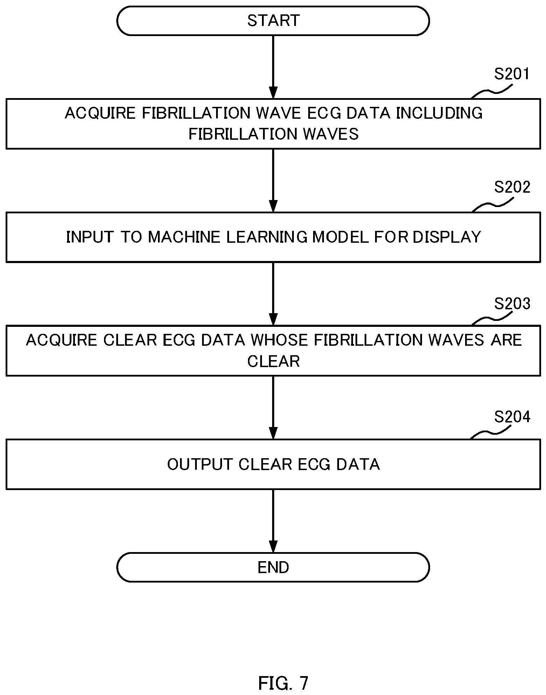

FIG. 7 is a flowchart showing a processing procedure for outputting the clear electrocardiogram data, performed by the output apparatus 3. This processing procedure starts when the first acquisition part 351 acquires, from the electrocardiograph 1, whole electrocardiogram data obtained by measuring the time variation of an action potential caused by the electric activity of the heart muscle.

The first acquisition part 351 acquires one or more pieces of fibrillation wave electrocardiogram data including fibrillation waves included in the acquired whole electrocardiogram data (S201). The second acquisition part 352 inputs one or more pieces of fibrillation wave electrocardiogram data to the machine learning part 33 for display that functions as a machine learning model for display (S202), and acquires clear electrocardiogram data which is output as the clear electrocardiogram data whose fibrillation waves are clear from the machine learning part 33 for display (S203). The output part 354 outputs the clear electrocardiogram data acquired by the second acquisition part 352 to the doctor's device 2 (S204), and ends the process.

### [Effect of the output apparatus of the present embodiment]

The second acquisition part 352 inputs the acquired plurality of pieces of fibrillation wave electrocardiogram data to the learned machine learning model for display, and acquires the clear electrocardiogram data output from the machine learning model for display as the clear electrocardiogram data whose fibrillation waves are clear. The output part 354 outputs the identified clear electrocardiogram data to the doctor's device 2. In this way, the output part 354 enables the doctor to grasp the waveform of the electrocardiogram, which is the basis for the doctor to determine that atrial fibrillation occurs in the patient's heart.

The present disclosure is explained based on the exemplary embodiments. The technical scope of the present disclosure is not limited to the scope explained in the above embodiments and it is possible to make various changes and modifications within the scope of the disclosure. For example, all or part of the apparatus can be configured with any unit which is functionally or physically dispersed or integrated. Further, new exemplary embodiments generated by arbitrary combinations of them are included in the exemplary embodiments. Further, effects of the new exemplary embodiments brought by the combinations also have the effects of the original exemplary embodiments.

### [Description of the reference numerals]

- 1: Electrocardiograph
- 2: Doctor's device
- 3: Output apparatus
- 31: Communication part
- 32: Machine learning part for determination
- 33: Machine learning part for display
- 34: Storage part
- 35: Control part
- 351: First acquisition part
- 352: Second acquisition part
- 353: Determination part
- 354: Output part
- 355: Accepting part
- 356: Generation part

## Claims

1. A program for causing a computer to execute the steps of:
acquiring one or more pieces of fibrillation wave electrocardiogram data including fibrillation waves included in whole electrocardiogram data obtained by measuring change over time of an action potential caused by electric activity of a heart muscle;
inputting the one or more pieces of fibrillation wave electrocardiogram data into a first machine learning model that classifies a plurality of pieces of electrocardiogram data including the fibrillation waves into (i) electrocardiogram data in which the fibrillation waves are clear and (ii) electrocardiogram data in which the fibrillation waves are unclear, and acquiring clear electrocardiogram data output from the first machine learning model as the electrocardiogram data in which the fibrillation waves are clear; and
outputting the clear electrocardiogram data.

2. The program according to claim 1, wherein
the acquiring the one or more pieces of fibrillation wave electrocardiogram data includes:
generating a plurality of pieces of divided electrocardiogram data obtained by dividing the whole electrocardiogram data;
inputting the generated plurality of pieces of divided electrocardiogram data to a second machine learning model that classifies the plurality of pieces of electrocardiogram data into (i) the electrocardiogram data including fibrillation waves and (ii) the electrocardiogram data not including fibrillation waves; and
acquiring one or more pieces of fibrillation wave electrocardiogram data output as the electrocardiogram data including the fibrillation waves output from the second machine learning model.

3. The program according to claim 1 or 2, wherein
the outputting includes outputting the clear electrocardiogram data in a state in which image data indicating that fibrillation waves are included is superimposed on a time domain between a plurality of R waves in the clear electrocardiogram data.

4. The program according to any one of claims 1 to 3, wherein
the outputting includes outputting the clear electrocardiogram data in which image data indicating that fibrillation waves are included is superimposed on a time domain within a predetermined time period before a timing at which a Q wave starts in the clear electrocardiogram data.

5. The program according to claim 4, wherein
the outputting includes outputting the clear electrocardiogram data in which the image data indicating that the fibrillation waves are included is superimposed on a time domain between a timing at which a T wave ends and a timing at which the Q wave starts in the clear electrocardiogram data.

6. The program according to any one of claims 1 to 5 further comprising:
determining whether there is a signal in a frequency range corresponding to fibrillation waves by performing a frequency analysis on the acquired clear electrocardiogram data, wherein
the outputting includes outputting the clear electrocardiogram data for which the determining determined that the signal in the frequency range corresponding to the fibrillation waves exists therein.

7. An output apparatus comprising:
a first acquisition part that acquires one or more pieces of fibrillation wave electrocardiogram data including fibrillation waves included in whole electrocardiogram data obtained by measuring change over time of an action potential caused by electric activity of a heart muscle; and
a second acquisition part that inputs the one or more pieces of fibrillation wave electrocardiogram data to a first machine learning model that classifies a plurality of pieces of electrocardiogram data including the fibrillation waves into (i) electrocardiogram data in which the fibrillation waves are clear and (ii) electrocardiogram data in which the fibrillation waves are unclear, and acquires clear electrocardiogram data output from the first machine learning model as the clear electrocardiogram data in which the fibrillation waves are clear; and
an output part that outputs the clear electrocardiogram data acquired by the second acquisition part.

8. A data processing method comprising the computer-implemented steps of:
acquiring one or more pieces of fibrillation wave electrocardiogram data including fibrillation waves included in whole electrocardiogram data obtained by measuring change over time of an action potential caused by electric activity of a heart muscle;
inputting the one or more pieces of fibrillation wave electrocardiogram data into a first machine learning model that classifies a plurality of pieces of electrocardiogram data including the fibrillation waves into (i) electrocardiogram data in which the fibrillation waves are clear and (ii) electrocardiogram data in which the fibrillation waves are unclear, and acquiring clear electrocardiogram data output from the first machine learning model as the electrocardiogram data in which the fibrillation waves are clear; and
outputting the clear electrocardiogram data.

9. The data processing method according to claim 8, further comprising, prior to acquiring the clear electrocardiogram data:
acquiring a plurality of pieces of learning electrocardiogram data which are a plurality of pieces of electrocardiogram data including fibrillation waves;
accepting an instruction to classify the plurality of pieces of learning electrocardiogram data into clear learning electrocardiogram data in which fibrillation waves are clear and unclear learning electrocardiogram data in which the fibrillation waves are unclear; and
generating a machine learning model that classifies a plurality of pieces of electrocardiogram data including the fibrillation waves into (i) electrocardiogram data in which the fibrillation waves are clear and (ii) electrocardiogram data in which the fibrillation waves are unclear by learning a plurality of pieces of the clear learning electrocardiogram data and a plurality of pieces of the unclear learning electrocardiogram data, both serving as training data.
